(19) Europäisches Patentamt
European Patent Office
Office européen des brevets

(11) **EP 4 311 491 A1**

(12) **EUROPEAN PATENT APPLICATION**

(43) Date of publication:
**31.01.2024 Bulletin 2024/05**

(21) Application number: **22186700.5**

(22) Date of filing: **25.07.2022**

(51) International Patent Classification (IPC):
**A61B 6/00** *(2024.01)*        **G01V 5/00** *(2024.01)*

(52) Cooperative Patent Classification (CPC):
**A61B 6/4241; A61B 6/481; A61B 6/482;
A61B 6/504; A61B 6/507;** G01N 23/087

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **NICKISCH, Hannes**
**Eindhoven (NL)**

• **HAASE, Hans Christian**
**Eindhoven (NL)**
• **GRASS, MICHAEL**
**5656AG Eindhoven (NL)**
• **SCHMITT, Holger**
**5656AG Eindhoven (NL)**
• **LANGZAM, Eran**
**Eindhoven (NL)**

(74) Representative: **Philips Intellectual Property &
Standards
High Tech Campus 52
5656 AG Eindhoven (NL)**

(54) **DETERMINING A VALUE OF A BLOOD FLOW PARAMETER**

(57)    A computer-implemented method of determining a value of a blood flow parameter for a vessel, is provided. The method includes: calculating from spectral attenuation data, and using a plurality of different techniques, a value of a blood flow parameter for the vessel; and providing the value of the blood flow parameter for the vessel based on the calculated values of the blood flow parameter.

Multi-channel
reconstructed images
$130_{1..n}$

Set of boundary conditions
$150_{1..i}$

**FIG. 4**

**Description**

TECHNICAL FIELD

The present disclosure relates to determining a value of a blood flow parameter for a vessel. A computer-implemented method, a computer program product, and a system are disclosed.

BACKGROUND

Various clinical investigations involve an assessment of blood flow in the vasculature. For instance, investigations for coronary artery diseases "CAD" often involve an assessment of blood flow in order to assess the amount of blood supplied to regions of the heart. In this regard, various blood flow parameters have been investigated, including blood flow velocity, blood pressure, the Fractional Flow Reserve "FFR", the instantaneous wave-free ratio "iFR", the Coronary Flow Reserve "CFR", the Thrombolysis in Myocardial Infarction "TIMI" flow grade, the Index of Microvascular Resistance "IMR", and the Hyperaemic Microvascular Resistance index "HMR".

Historically, values for blood flow parameters such as those mentioned above have been measured using invasive devices such as a pressure-wire. For instance, a value for the Fractional Flow Reserve "FFR" is often determined in order to assess the impact of a stenosis on delivery of oxygen to the heart muscle in investigations for CAD. The FFR is defined by the ratio $P_d/P_a$, wherein $P_d$ represents a distal pressure at a distal position with respect to the stenosis, and $P_a$ represents a proximal pressure with respect to the stenosis. The FFR is typically calculated with values for $P_d$ and $P_a$ that are averaged over the cardiac cycle. FFR values above 0.8 are typically regarded as clinically insignificant, and values below 0.8 are typically considered to represent increased clinical significance. Historically, values for these pressures have been determined by positioning an invasive device, such as a pressure wire, at the respective positions in the vasculature.

More recently, angiographic techniques have been developed for determining the values of blood flow parameters, including the FFR. According to fluid flow theory, pressure changes are, among other effects, linked to changes in fluid velocity. In the example of the FFR, angiographic images of an injected contrast agent are analyzed in order to determine the blood flow velocity. The FFR is then calculated by using a haemodynamic model to estimate pressure values in the vessel from the blood flow velocity. Thus, a value for the FFR, as well as other blood flow parameters may be determined angiographically.

A known angiographic technique for measuring blood flow velocity is to sample the intensity of an injected contrast agent in computed tomography "CT" angiographic image data over time, and to apply a mathematical model to the sampled data. In this regard, a technique for sampling reconstructed CT images in order to determine blood flow velocity is disclosed in a document by Barfett, J. J., et al., "Intra-vascular blood velocity and volumetric flow rate calculated from dynamic 4D CT angiography using a time-of-flight technique", Int J Cardiovasc Imaging, 2014, 30:1383-1392. A technique for sampling raw, i.e., "projection" CT data in order to determine blood flow velocity is disclosed in a document by Prevrhal, S. et al., "CT Angiographic Measurement of Vascular Blood Flow Velocity by Using Projection Data", Radiology, Vol. 261: Number 3 - December 2011, pages 923 - 929.

Some known angiographic techniques for determining the value of blood flow parameters use a computational fluid dynamics "CFD" model. In this regard, a technique for determining an FFR value is disclosed in a document by Taylor. C. A., et al., "Computational Fluid Dynamics Applied to Cardiac Computed Tomography for Noninvasive Quantification of Fractional Flow Reserve: Scientific Basis", JACC, Vol. 61, No. 22, 2013. Another document describing an angiographic technique for measuring blood flow parameters using a complex fluid dynamics "CFD" model is the document by Koo, B. K., et al., "Diagnosis of Ischemia-Causing Coronary Stenoses by Noninvasive Fractional Flow Reserve Computed From Coronary Computed Tomographic Angiograms: Results From the Prospective Multicenter DISCOVER-FLOW (Diagnosis of Ischemia-Causing Stenoses Obtained Via Noninvasive Fractional Flow Reserve) Study", JACC, Vol. 58, No. 19, 2011. Another technique is disclosed in a document by Kim, H. J., et al., "Patient-Specific Modeling of Blood Flow and Pressure in Human Coronary Arteries", Annals of Biomedical Engineering, Vol. 38, No. 10, October 2010, pp. 3195-3209.

Another angiographic technique for measuring blood flow parameters involves the use of a lumped parameter model. A lumped parameter model can be used to represent blood flow in a vascular region as an electrical circuit; a volumetric flow rate of the blood being represented as an electrical current, a pressure of the blood being represented as a voltage, and a blood volume being represented by an electrical charge. In a lumped parameter model, resistance to blood flow is represented by a linear or nonlinear electrical resistor, vessel wall compliance is represented by a capacitance, and blood inertia is represented by an inductance. A machine learning-based approach for setting the values of linear and nonlinear resistors in a static lumped parameter model based on angiographic geometric measurements of the vessels in the vascular region is disclosed in a document by Nickisch, H., et al., "Learning Patient-Specific Lumped Models for Interactive Coronary Blood Flow Simulations", MICCAI 2015, Part II, LNCS 9350, pp. 433 - 441, 2015, and also in the document WO 2016/001017. Values for the blood flow rate and pressure in the model are then solved in order to determine a value for the FFR for a vessel. As compared to computational fluid dynamics "CFD" models, the use of a lumped parameter model to calculate values of blood flow parameters offers the advantages of a reduction in both computational burden, and model complexity.

In general, the basis for providing accurate values for blood flow parameters from angiographic image data is the correct anatomical delineation, or segmentation, of the vessel lumen. This image-processing task may be performed manually, which is both time-consuming and risks introducing user-bias, or automatically, and in which case depends on the technique used to segment the angiographic data. As a consequence, there can be a variability in the values of angiographically-derived values of blood flow parameters.

Thus, there remains room to improve the reliability of angiographic measurements of blood flow parameters such as blood flow velocity, blood pressure, the FFR, the iFR, the CFR, the TIMI flow grade, the IMR, and the HMR.

SUMMARY

According to one aspect of the present disclosure, a computer-implemented method of determining a value of a blood flow parameter for a vessel, is provided. The method includes:

receiving spectral attenuation data representing an injected contrast agent within a vascular region including the vessel, the spectral attenuation data defining X-ray attenuation within the vascular region in a plurality of different energy intervals $DE_{1..m}$;

calculating, from the spectral attenuation data, and using a plurality of different techniques, a value of a blood flow parameter for the vessel; and

providing the value of the blood flow parameter for the vessel based on the calculated values of the blood flow parameter.

As compared to the use of conventional X-ray attenuation data, the use of spectral attenuation data in the above method to calculate the value of the blood flow parameter facilitates improved separation between the attenuation arising from the injected contrast agent, and attenuation arising from other media that may be present in the vascular region. Attenuation arising from other media, such as for example calcium lesions in a blood vessel, might otherwise be interpreted as contrast agent, and consequently degrade the accuracy of the calculated blood flow parameter. The use of spectral attenuation data also helps to reduce image artifacts arising from media other than the contrast agent, and which may likewise be misinterpreted as contrast agent, and likewise degrade the accuracy of the calculated blood flow parameter. For example, calcium lesions can also cause "calcium blooming" in conventional CT data, which results in image intensity values that risk being mis-interpreted as the result of attenuation arising from the contrast agent. Thus, the use of spectral attenuation data facilitates a more accurate calculation of blood flow parameter values. However, the values of blood flow parameters that are calculated using spectral attenuation data, can still be subject to variation. For instance, the choice of energy intervals to use to reconstruct an image, from which image the value of the blood flow parameter is calculated, can also affect the calculated value of the blood flow parameter. The values of model parameters, such as the boundary conditions, of a blood flow computation algorithm that is used to calculate the blood flow parameter, can also affect its value. Similarly, the choice of segmentation algorithm that is used to segment reconstructed image data, and from which the value of the blood flow parameter is calculated, and also the choice of blood flow computation algorithm that is used to calculate the value of the blood flow parameter, can also affect its value. In the above method, since a value of a blood flow parameter for the vessel is calculated using a plurality of different techniques, and the value of the blood flow parameter is provided based on the calculated values, the reliability of the provided value of the blood flow parameter, is improved.

Further aspects, features, and advantages of the present disclosure will become apparent from the following description of examples, which is made with reference to the accompanying drawings.

BRIEF DESCRIPTION OF THE DRAWINGS

Fig. 1 is a flowchart illustrating an example of a computer-implemented method of determining a value of a blood flow parameter for a vessel, in accordance with some aspects of the present disclosure.

Fig. 2 is a schematic diagram illustrating an example of a system 200 for determining a value of a blood flow parameter for a vessel, in accordance with some aspects of the present disclosure.

Fig. 3 is a schematic diagram illustrating a heart, including an example of a vessel 110, in accordance with some aspects of the present disclosure.

Fig. 4 is a schematic diagram illustrating an example of a computer-implemented method of determining a value of a blood flow parameter for a vessel, in accordance with some aspects of the present disclosure.

Fig. 5 is a graph illustrating the dependence of the mass attenuation coefficient with X-ray energy for two example materials, iodine and water.

Fig. 6 is a schematic diagram illustrating an example of a lumped parameter model 140 including a set of model parameters $150_1$, in accordance with some aspects of the present disclosure.

Fig. 7 is a schematic diagram illustrating an example of an operation of generating S140 a contrast-adjusted reconstructed image, in accordance with some aspects of the present disclosure.

## DETAILED DESCRIPTION

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer implemented method, may be implemented in a computer program product, and in a system, in a corresponding manner.

In the following description, reference is made to a method of determining a value of a blood flow parameter for a vessel. The vessel is disposed within a vascular region. In some examples, the vascular region is the heart. For instance, in some examples, the vessel is a coronary artery. However, it is to be appreciated that the vessel may in general be an artery or a vein. More generally, the method may be used to measure a blood flow parameter for a vessel in another part of the body than the heart. For example, the region(s) of interest may alternatively be a vessel that is located in a peripheral region, such as in the leg, the arm, the brain, and so forth.

Reference is also made herein to examples in which the calculated blood flow parameter is an FFR value for a vessel. However, it is to be appreciated that this serves only as an example, and that the method and system disclosed herein may alternatively be used to calculate values for other blood flow parameters, such as, and without limitation, a blood flow velocity, a blood pressure, a blood flow transit time, a volumetric blood flow value, an iFR value, a CFR value, a TIMI flow grade, an IMR value, and an HMR value, a hyperaemic stenosis resistance "HSR" value, a zero flow pressure "ZFP" value, and an instantaneous hyperaemic diastolic velocity-pressure slope "IHDVPS" value.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray™ and DVD.

As mentioned above, there remains room to improve the reliability of angiographic measurements of blood flow parameters such as blood flow velocity, blood pressure, the FFR, the iFR, the CFR, the TIMI flow grade, the IMR, and the HMR.

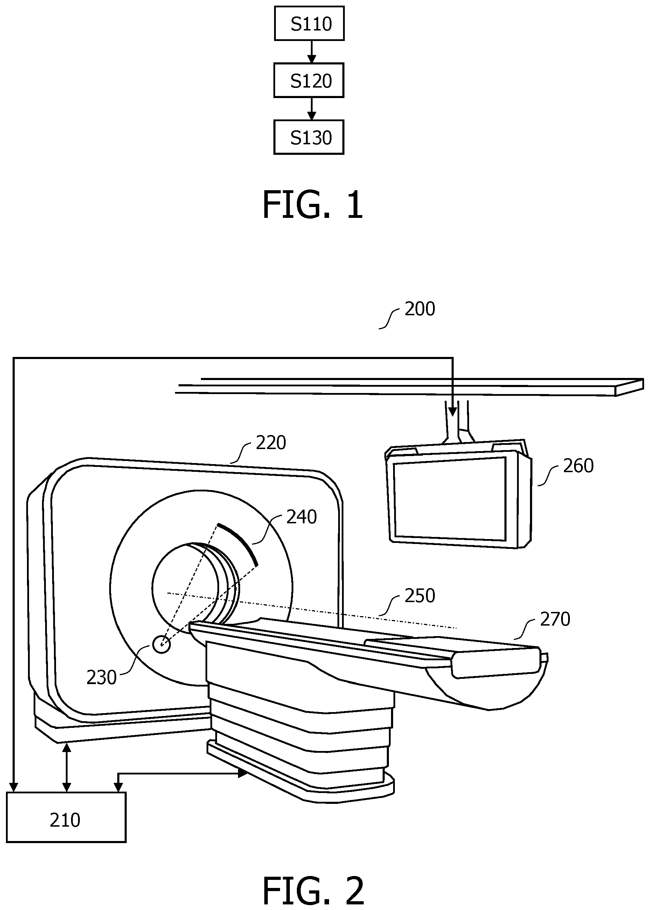

Fig. 1 is a flowchart illustrating an example of a computer-implemented method of determining a value of a blood flow parameter for a vessel, in accordance with some aspects of the present disclosure. Fig. 2 is a schematic diagram illustrating an example of a system 200 for determining a value of a blood flow parameter for a vessel, in accordance with some aspects of the present disclosure. Operations described in relation to the method illustrated in Fig. 1, may also be performed by the system 200 illustrated in Fig. 2, and vice versa. With reference to Fig. 1, the computer-implemented method of determining a value of a blood flow parameter for a vessel 110, includes:

receiving S110 spectral attenuation data 120 representing an injected contrast agent within a vascular region including the vessel 110, the spectral attenuation data defining X-ray attenuation within the vascular region in a plurality of different energy intervals $DE_{1..m}$;

calculating S120, from the spectral attenuation data 120, and using a plurality of different techniques, a value of a blood flow parameter for the vessel 110; and

providing S130 the value of the blood flow parameter for the vessel 110 based on the calculated values of the blood flow parameter.

As compared to the use of conventional X-ray attenuation data, the use of spectral attenuation data in the above method to calculate the value of the blood flow parameter facilitates improved separation between the attenuation arising from the injected contrast agent, and attenuation arising from other media that may be present in the vascular region. Attenuation arising from other media, such as for example calcium lesions in a blood vessel, might otherwise be interpreted as contrast agent, and consequently degrade the accuracy of the calculated blood flow parameter. The use of spectral attenuation data also helps to reduce image artifacts arising from media other than the contrast agent, and which may likewise be misinterpreted as contrast agent, and likewise degrade the accuracy of the calculated blood flow parameter. For example, calcium lesions can also cause "calcium blooming" in conventional CT data, which results in image intensity values that risk being mis-interpreted as the result of attenuation arising from the contrast agent. Thus, the use of spectral attenuation data facilitates a more accurate calculation of blood flow parameter values. However, blood flow parameters that are calculated using spectral attenuation data, can still be subject to variation. For instance, the choice of energy intervals to reconstruct an image, and from which the value of the blood flow parameter is calculated, can also affect the calculated value of the blood flow parameter. The values of model parameters, such as the boundary conditions, of a blood flow computation algorithm that is used to calculate the blood flow parameter, can also affect its value. Similarly, the choice of segmentation algorithm that is used to segment reconstructed image data, and from which the value of the blood flow parameter is calculated, and also the choice of blood flow computation algorithm that is used to calculate the value of the blood flow parameter, can also affect its value. In the above method, since a value of a blood flow parameter for the vessel is calculated using a plurality of different techniques, and the value of the blood flow parameter is provided based on the calculated values, the reliability of the provided value of the blood flow parameter, is improved.

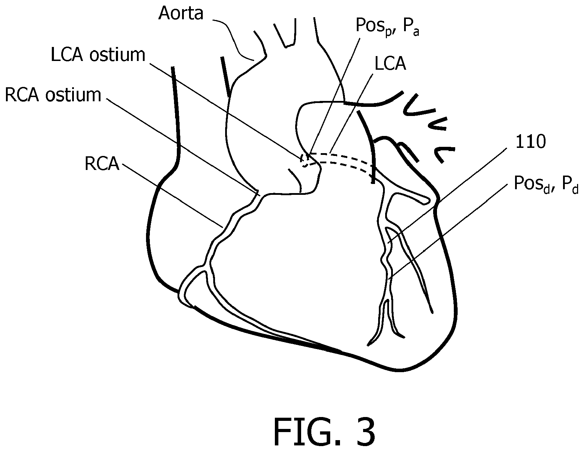

The above method is also described with reference to Fig. 3, which is a schematic diagram illustrating a heart, including an example of a vessel 110, in accordance with some aspects of the present disclosure. The heart illustrated in Fig. 3 is labelled with the left coronary artery, LCA, the right coronary artery, RCA, and the LCA ostium and RCA ostium that respectively define the openings of these arteries in the aorta. The operations described above with reference to Fig. 1 may be performed in order to determine a value of a blood flow parameter in a cardiac vascular region. For example, they may be used to determine a value of a blood flow parameter in the vessel 110 of the heart illustrated in Fig. 3. The blood flow parameter may be an FFR value, for example. The FFR value may be calculated for a distal position $Pos_d$ in the left coronary artery using the equation:

$$FFR = P_d/P_a \qquad\qquad \text{Equation 1}$$

The FFR value may be calculated using Equation 1 by determining a value for the blood pressure $P_d$ at the distal position $Pos_d$, and a value for the pressure $P_a$ at a proximal position $Pos_p$, which in this example is taken as the ostium of the left coronary artery.

Returning to Fig. 1, in the operation S110 spectral attenuation data 120 is received. The spectral attenuation data 120 represents an injected contrast agent within a vascular region including the vessel 110, and defines X-ray attenuation within the vascular region in a plurality of different energy intervals $DE_{1..m}$.

The spectral attenuation data 120 that is received in the operation S110 may in general be projection X-ray data, i.e. 2D data that can be used to generate a 2D image, or it may be volumetric X-ray data, i.e. 3D data that can be used to generate a 3D image. In the latter case, the data may be raw data, i.e. data that has not yet been reconstructed into a 3D image, or it may be image data, i.e. data that represents a reconstructed 3D image.

The spectral attenuation data 120 may be received by the one or more processors 210 illustrated in Fig. 2. The spectral attenuation data 120 may be received via any form of data communication, including wired, optical, and wireless communication. By way of some examples, when wired or optical communication is used, the communication may take place via signals transmitted on an electrical or optical cable, and when wireless communication is used, the communication may take place via RF or optical signals. In general, the one or more processors 210 may receive the spectral attenuation data 120 from a spectral X-ray projection imaging system, or from a spectral CT imaging system, or from another source, such as a computer readable storage medium, the Internet, or the Cloud, for example.

With continued reference to Fig. 1, the spectral attenuation data 120 that is received in the operation S110 represents an injected contrast agent within a vascular region including the vessel 110. As described above with reference to Fig. 3, the spectral attenuation data 120 may for instance represent an injected contrast agent in a cardiac region. In this regard, the spectral attenuation data that is received in the operation S110 may be generated subsequent to the injection of a contrast agent into the vasculature. The contrast agent may include a substance such as iodine, or a Lanthanide such as gadolinium, or another substance that provides visibility of a blood flow into which the contrast agent is injected.

With continued reference to Fig. 1, the spectral attenuation data 120 that is received in the operation S110 may in general be generated by a spectral X-ray imaging system, i.e., it may be generated by a spectral CT imaging system or by a spectral X-ray projection imaging system.

A spectral CT imaging system generates spectral attenuation data whilst rotating, or stepping, an X-ray source-detector arrangement around an imaging region. Examples of spectral CT imaging systems include cone beam spectral CT imaging systems, photon counting spectral CT imaging systems, dark-field spectral CT imaging systems, and phase contrast spectral CT imaging systems. By way of an example, the spectral attenuation data 120 may be generated by the Spectral CT 7500 that is marketed by Philips Healthcare, Best, The Netherlands.

An example of a spectral CT imaging system 220 that may be used to generate the spectral attenuation data 120 that is received in the operation S110, is illustrated in Fig. 2. The spectral CT imaging system 220 illustrated in Fig. 2 includes an X-ray source 230 and the X-ray detector 240. The X-ray source 230 and X-ray detector 240 are mechanically coupled to a gantry (not illustrated in Fig. 2). In operation, the X-ray source 230 and X-ray detector 240 are rotated by means of the gantry around an axis of rotation 250 whilst acquiring spectral attenuation data 120 that defines X-ray attenuation in a region of interest disposed in an imaging region of the imaging system 220. The spectral attenuation data that is obtained from multiple rotational angles around the axis of rotation 250, or more specifically, volumetric X-ray data, i.e. 3D data, may subsequently be reconstructed into a volumetric image.

As mentioned above, the spectral attenuation data that is received in the operation S110 may alternatively be generated by a spectral X-ray projection imaging system. Spectral X-ray projection imaging systems typically include a support arm such as a so-called "C-arm" that supports an X-ray source and an X-ray detector. Spectral X-ray projection imaging systems may alternatively include a support arm with a different shape to this example, such as an O-arm, for example. Spectral X-ray projection imaging systems typically generate projection data with the support arm held in a static position with respect to an imaging region during the acquisition of image data. Thus, spectral X-ray projection imaging systems may be used to acquire projection X-ray data, i.e. 2D data. However, spectral X-ray projection imaging systems may also acquire volumetric X-ray data, i.e. 3D data, by acquiring projection X-ray data whilst rotating their support arm around an axis of rotation. Image reconstruction techniques may then be used to reconstruct the projection X-ray data that is obtained from multiple rotational angles around the axis of rotation into a volumetric image in a similar manner to a spectral CT imaging system. Thus the spectral attenuation data that is received in the operation S110 may alternatively be generated by a spectral X-ray projection imaging system.

The spectral attenuation data 120 that is received in the operation S110 defines X-ray attenuation within the vascular region in a plurality of different energy intervals $DE_{1..m}$. In general there may be two or more energy intervals; i.e. $m$ is an integer, and $m \geq 2$. The ability to generate data that defines X-ray attenuation data in multiple different energy intervals $DE_{1..m}$ distinguishes a spectral X-ray imaging system from a conventional X-ray imaging system. By processing the data from the multiple different energy intervals, a distinction can be made between media that have similar X-ray attenuation values when measured within a single energy interval, and which would be indistinguishable from attenuation data generated by a conventional X-ray imaging system. In this regard, various different configurations of spectral X-ray imaging systems may be used to generate the spectral attenuation data that is received in the operation S110, some of which are described with reference to Fig. 2.

With reference to the example spectral CT imaging system 220 illustrated in Fig. 2, in general, the X-ray source 230 may be provided by multiple monochromatic sources, or by one or more polychromatic sources, and the X-ray detector 240 may include: a common detector for detecting multiple different X-ray energy intervals, or multiple detectors wherein each detector detects a different X-ray energy interval $DE_{1..m}$, or a multi-layer detector in which X-rays having energies within different X-ray energy intervals are detected by corresponding layers, or a photon counting detector that bins detected X-ray photons into one of multiple energy intervals based on their individual energies. In a photon counting detector, the relevant energy interval may be determined for each received X-ray photon by detecting the pulse height induced by electron-hole pairs that are generated in response to the X-ray photon's absorption in a direct-conversion material.

Various configurations of the aforementioned X-ray sources and X-ray detectors may be used in a spectral X-ray imaging system to generate spectral attenuation data defining X-ray attenuation in a plurality of different energy intervals $DE_{1..m}$. In general, a discrimination between different X-ray energy intervals may be provided at the X-ray source 230 by temporally switching the X-ray tube potential of a single X-ray source 230, i.e. by "rapid kVp switching", or by temporally switching, or filtering, the emission of X-rays from multiple X-ray sources 230. In such configurations, a common X-ray detector 240 may be used to detect X-rays across multiple different energy intervals, attenuation data for each of the energy intervals $DE_{1..m}$ being generated in a time-sequential manner. Alternatively, a discrimination between different X-ray energy intervals $DE_{1..m}$ may be provided at the X-ray detector 240 by using a multi-layer detector, or a photon counting detector. Such detectors can detect X-rays from multiple X-ray energy intervals $DE_{1..m}$ near-simultaneously, and thus there is no need to perform temporal switching at the X-ray source 230. Thus, a multi-layer X-ray detector 240, or a photon counting X-ray detector 240, may be used in combination with a polychromatic X-ray source 230 to generate spectral attenuation data in a plurality of different energy intervals $DE_{1..m}$.

Other combinations of the aforementioned X-ray sources 230 and X-ray detectors 240 may alternatively be used in a spectral X-ray imaging system to provide the desired spectral attenuation data in a plurality of different energy intervals $DE_{1..m}$. For example, in another configuration, the need to sequentially switch different X-ray sources 230 emitting X-rays in different energy intervals may be obviated by mounting X-ray source-detector pairs to a gantry at rotationally-offset positions around the axis of rotation 250. In this configuration, each source-detector pair operates independently, and a separation between the spectral attenuation data for the different energy intervals $DE_{1..m}$ is facilitated by virtue of the rotational offsets of the source-detector pairs. Improved separation between the spectral attenuation data for the different energy intervals $DE_{1..m}$ may be achieved with this configuration by applying an energy-selective filter to the X-ray detector(s) 240 in order to reduce the effects of X-ray scatter.

In yet another configuration, spectral attenuation data in a plurality of different energy intervals $DE_{1..m}$ may be provided by intercepting the X-ray beam in a conventional X-ray imaging system with one or more spectral filters. For example, a single filter may be mechanically moved into, and out of, the X-ray beam generated by a polychromatic X-ray source 230 in order to temporally control the spectrum of X-rays detected by an X-ray detector 240. The filter may for instance transmit only a portion of the spectrum of X-rays emitted by the polychromatic X-ray source 230, and thereby provide spectral attenuation data for a first energy interval when the filter intercepts the beam. When the filter is removed from the beam, spectral attenuation data is provided for a second energy interval, e.g. for the complete spectrum emitted by the polychromatic X-ray source. In so doing, attenuation data for the energy intervals $DE_{1..m}$ is generated in a time-sequential manner. Multiple filters, each with a different X-ray transmission spectrum, may be sequentially switched into, and out of, the X-ray beam in order to increase the number of energy intervals.

Returning to Fig. 1, in the operation S120, a value of a blood flow parameter for the vessel 110 is calculated using a plurality of different techniques. By way of some examples, the blood flow parameter may be a blood flow velocity, a blood pressure, an FFR value, or an iFR value. The different techniques that are used in the operation S120 may for example include the use of different techniques to reconstruct the received spectral attenuation data, different segmentation algorithms to segment an image that is reconstructed from the spectral attenuation data, the use of different blood flow computation algorithms to calculate a value of the blood flow parameter, and the use of different sets of model parameters for a blood flow computation algorithm. Further detail on these techniques is provided below. A combination of these, and other, techniques may also be used to provide different values for the blood flow parameter.

With continued reference to Fig. 1, in the operation S130, the value of the blood flow parameter for the vessel 110 is provided based on the calculated values of the blood flow parameter. In one example, the operation S130 may include providing the value of the blood flow parameter for the vessel 110 as a weighted average of the individual calculated values. By providing the value of the blood flow parameter as a weighted average, the reliability of the measurement is improved. In another example, a weighted average is calculated, and outlier values are removed prior to calculating the weighted average. In this example, the operation of providing S130 the value of the blood flow parameter for the vessel 110 based on the calculated values of the blood flow parameter comprises:

analyzing the calculated values of the blood flow parameter to identify outlier values; and
providing the value of the blood flow parameter for the vessel 110 as a weighted average of the calculated values that are not identified as outlier values.

The exclusion of outlier values from the weighted average that is calculated in this example has the effect of reducing the impact of potentially inaccurate values of the blood flow parameter on the provided value of the blood flow parameter. Thus, it reduces the amount of variation in the calculated values of the blood flow parameter, and consequently improves the reliability of the provided value.

In this example, the analysis of the calculated values of the blood flow parameter to identify outlier values may include performing a statistical analysis on the calculated values of the blood flow parameter. For instance, the mean of the calculated values may be determined, and values that deviate from the mean by more than a predetermined amount may be identified as outlier values. By way of another example, a standard deviation may be determined for the calculated values, and values that lie outside e.g. two standard deviations of the mean may be identified as outlier values.

By way of another example, the RANSAC method may be used to identify, and to exclude, outlier values from the weighted average.

Since the value of the blood flow parameter for the vessel 110 that is provided in the operation S130 is determined based on the calculated values of the blood flow parameter, and which have been calculated using different techniques, the reliability of the provided value of the blood flow parameter for the vessel is improved.

The computer-implemented method described above may include one or more additional operations.

In one example, the method described with reference to Fig. 1 may also include outputting a value representing a variation in the calculated values for the blood flow parameter. The outputting of the value representing the variation provides an indication of the level confidence of the result; a small amount of variation being indicative of high confidence, and vice-versa. A measure of the variation may be outputted numerically, or graphically, such as via error bars, for example.

In another example, the operations of calculating S120 a value of a blood flow parameter for the vessel 110, and providing S130 the value of the blood flow parameter for the vessel 110, are performed at a plurality of positions along the vessel.

By way of an example, FFR values for a vessel may be provided for multiple positions along a vessel, and consequently used by a physician to assess the severity of a stenosis along the vessel.

As mentioned above, the operation of calculating S120 a value of a blood flow parameter for the vessel 110 using a plurality of different techniques may be performed in various ways.

In one example, the calculating S120 operation includes reconstructing the received spectral attenuation data 120 using a plurality of different image reconstruction techniques to provide a plurality of corresponding reconstructed images $130_{1..n}$, and calculating a value of the blood flow parameter from each of the reconstructed images.

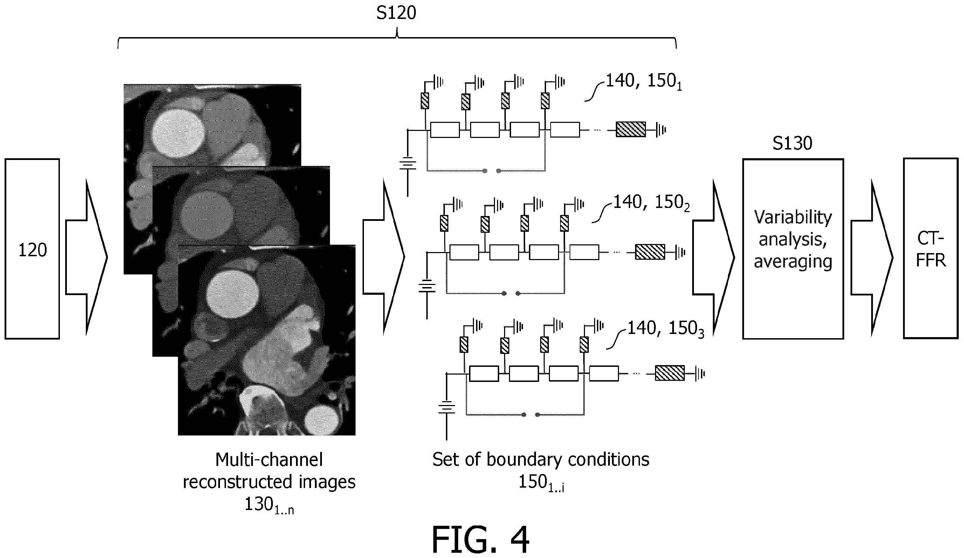

This example is described with reference to Fig. 4, which is a schematic diagram illustrating an example of a computer-implemented method of determining a value of a blood flow parameter for a vessel, in accordance with some aspects of the present disclosure. The spectral attenuation data 120 that is received in the operation S110 described above, is illustrated on the left-hand side of Fig. 4. The spectral attenuation data 120 in this example is volumetric X-ray data that is generated by a spectral CT imaging system. In the operation S120, the spectral attenuation data 120 is reconstructed using a plurality of different image reconstruction techniques to provide a plurality of corresponding reconstructed images $130_{1..n}$, as illustrated in Fig. 4. In this example, the reconstructed images may be reconstructed by:

applying different material decomposition algorithms to the spectral attenuation data 120; and/ or
reconstructing spectral attenuation data 120 comprising different selections of the energy intervals $DE_{1..m}$.

The resulting reconstructed images represent the injected contrast agent in the vascular region. The injected contrast agent may include a material such as iodine, or gadolinium. Such materials are often used as contrast agents in view of their attenuation at the X-ray energies used in diagnostic X-ray imaging systems. By reconstructing an image representing the injected contrast agent from spectral attenuation data, more reliable data on the flow of the injected contrast agent may be obtained, as compared to the use of conventional X-ray attenuation data.

In addition to the attenuation arising from the contrast agent, the spectral attenuation data may also represent attenuation arising from one or more background materials such as fat, water, bone, soft tissue, vessel calcification, air, and metals such as gold, titanium, tungsten, and platinum. Such materials are often also present in the vicinity of vessels, and therefore attenuation arising from these materials may also be represented in the spectral attenuation data. For example, when imaging the cardiac vasculature, bone, in the form of portions of the spine, or ribs are often within the field of view of a spectral CT imaging system. Likewise, fiducial markers, implanted medical devices, and interventional devices are typically formed from metals such as those mentioned above, and attenuation arising from these materials may also be captured in the spectral attenuation data. The reconstructed images that are generated in the operation S 120 may therefore also represent one or more materials other than the contrast agent, such as these.

One example of a material decomposition technique that may be used in the operation S120 in order to reconstruct an image representing the injected contrast agent from the spectral attenuation data, is disclosed in a document by Brendel, B. et al., "Empirical, projection-based basis-component decomposition method", Medical Imaging 2009, Physics of Medical Imaging, edited by Ehsan Samei and Jiang Hsieh, Proc. of SPIE Vol. 7258, 72583Y. Another suitable material decomposition technique is disclosed in a document by Roessl, E. and Proksa, R., "K-edge imaging in X-ray computed tomography using multi-bin photon counting detectors", Phys Med Biol. 2007 Aug 7, 52(15):4679-96. Another suitable material decomposition technique is disclosed in the published PCT patent application WO/2007/034359 A2. Another suitable material decomposition technique is disclosed in a document by Silva, A. C., et al., "Dual-energy (spectral) CT: applications in abdominal imaging", RadioGraphics 2011; 31(4):1031-1046.

Thus, in the operation S120, multiple different material decomposition algorithms such as these may be applied to the spectral attenuation data 120 in order to reconstruct an image representing the injected contrast agent.

In general, the X-ray attenuation spectrum of a material includes a contribution from Compton scatter and a contribution from the Photo-electric effect. The attenuation due to Compton scatter is relatively similar for different materials, whereas the attenuation from the Photo-electric effect is strongly material-dependent. Both Compton scatter and the Photo-electric effect exhibit an energy dependence, and it is this effect that is exploited by material decomposition techniques in order to distinguish between different materials.

In general, material decomposition algorithms operate by decomposing the attenuation spectrum of an absorbing medium into contributions from an assumed set of "basis" materials. The energy-dependent X-ray attenuation of the assumed basis materials is typically modelled as a combination of absorption from Compton scatter, and the Photo-electric effect. Some materials also have a k-absorption edge "k-edge" energy that is within the energy range used by diagnostic X-ray imaging systems, and this effect may also be exploited in order to distinguish between different materials. The spectral decomposition algorithm then seeks to estimate an amount of each of the basis materials that is required to produce the measured amount of X-ray attenuation at the two or more energy intervals. Water and iodine are examples of basis materials that are often separated in clinical practice using a so-called two-material decomposition algorithm. Non-fat soft tissue, fat, and iodine, are examples of basis materials that are often separated in clinical practice using a three-material decomposition algorithm.

As mentioned above, if the k-absorption edge "k-edge" energy of any of the basis materials is within the energy range used by diagnostic X-ray imaging systems, i.e., approximately 30 - 120 keV, this can be exploited in order to help identifying the contribution of a basis material to X-ray attenuation. The k-edge energy for a material is defined as the minimum energy required for the Photo-electric event to occur with a k-shell electron. The k-edge occurs at a characteristic energy for each material. The k-edge energy for a material is marked by a sharp increase in its X-ray attenuation spectrum at X-ray energies corresponding to the k-edge energy value. The k-edge energies of many materials that are present in the human body are too low to be detected in a diagnostic X-ray imaging system. For example, the k-edge energies of hydrogen, carbon, oxygen, and nitrogen are at energies that are less than 1 keV. However, materials such as iodine (k-edge = 33.2 keV), gadolinium (50.2 keV), gold (80.7 keV), platinum (78.4 keV), tantalum (67.4 keV), holmium (55.6 keV), and molybdenum (k-edge = 20.0 keV) have k-edge energy values that permit their distinction in spectral CT projection data acquired from diagnostic X-ray imaging systems.

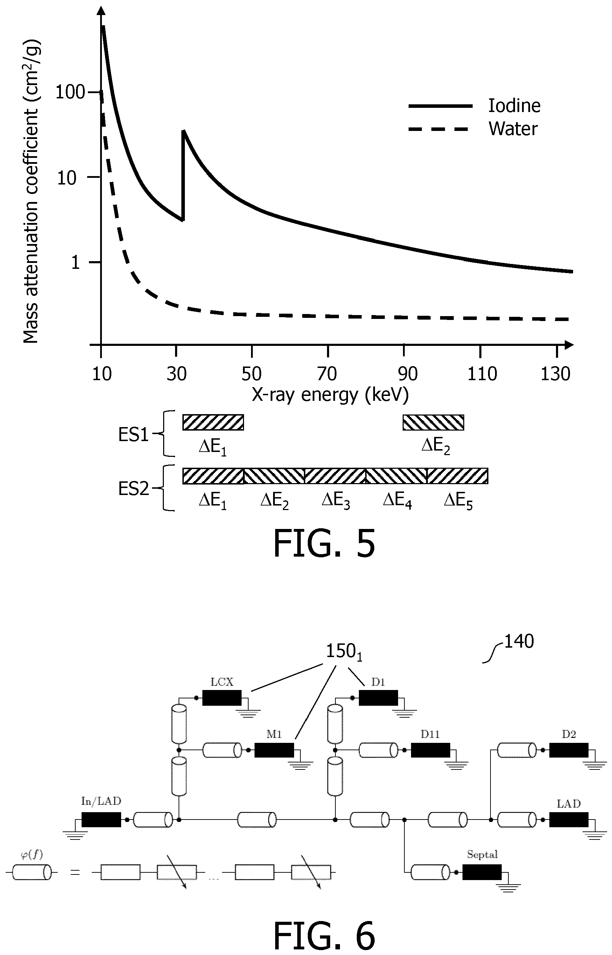

By way of an example, Fig. 5 is a graph illustrating the dependence of the mass attenuation coefficient with X-ray energy for two example materials, iodine and water. The mass attenuation coefficient represented in Fig. 5 represents the X-ray attenuation. The sharp increase in X-ray attenuation for iodine at 33.2 keV facilitates a separation between the contributions of each of these materials in a combined attenuation spectrum that includes attenuation arising from both of these materials. For instance, the X-ray attenuation arising from iodine and water may be separated by using a first selection of energy intervals, ES1, with one energy interval $DE_1$ of the spectral attenuation data close to the k-edge energy of 33.2 keV, and another energy interval $DE_2$ significantly above the k-edge energy.

In the operation S120, multiple different material decomposition algorithms may be used to reconstruct separate images representing the injected contrast agent, wherein a value of the blood flow parameter is then calculated from each of the reconstructed images in order to determine the provided value of the blood flow parameter. In this regard, the multiple different material decomposition algorithms that are used may be provided by the material decomposition algorithms cited above. Alternatively, or additionally, in the operation S120, different selections of the energy intervals $DE_{1..m}$ may be used to reconstruct separate images representing the injected contrast agent, wherein a value of the blood flow parameter is calculated from each of these reconstructed images. In this regard, an example of a selection of energy intervals, ES1, is illustrated in Fig. 5 for generating a reconstructed image by separating iodine from water. An alternative selection of energy intervals for separating iodine from water, could be provided by changing the width, or the central energy, of the energy intervals $DE_1$ and/ or $DE_2$. A different selection of energy intervals could also be provided as illustrated by the second selection of energy intervals ES2 in Fig. 5, and in which a different number of energy intervals and different ranges of the energy intervals, i.e. energy intervals $DE_{1..5}$, are used to separate iodine from water. Having selected the energy intervals, a material decomposition algorithm such as one of those cited above may be used in the operation S120 to reconstruct each of the images representing the injected contrast agent.

With continued reference to the example illustrated in Fig. 4, in the operation S120, after having reconstructed multiple images that each represent the injected contrast agent, a value of the blood flow parameter is determined for each of the reconstructed images. This operation is illustrated in the central portion of Fig. 4, and in which a blood flow computation algorithm 140 is used to determine a value of a blood parameter from each of the reconstructed images $130_{1..3}$. In the example illustrated in Fig. 4, the blood flow computation algorithm 140 includes a lumped parameter model. A lumped parameter model can be used to represent the blood flow within the vascular region as an electrical circuit; a volumetric flow rate of the blood in the vascular region being represented in the electrical circuit by an electrical current, and a pressure of the blood in the vascular region being represented in the electrical circuit by a voltage. The lumped parameter model is described in more detail below.

Instead of using a lumped parameter model to calculate a value of the blood flow parameter from each of the reconstructed images in the operation S120, other techniques may alternatively be used. These include the time of flight approach for sampling reconstructed CT images in order to determine blood flow velocity disclosed the document by Barfett, J. J., et al., cited above, the technique for sampling raw, i.e. "projection" CT data in order to determine blood flow velocity disclosed in the document by Prevrhal, S. et al., cited above, and also computational fluid dynamic "CFD" techniques such as the technique for determining an FFR value disclosed in the document by Taylor. C. A., et al., cited above, and in the document by Koo, B. K., et al., cited above, and in the document by Kim, H. J., et al., cited above.

With continued reference to Fig. 4, having calculated a value of the blood flow parameter from each of the reconstructed images using a blood flow computation algorithm in the operation S120, the method illustrated in Fig. 4 continues with the operation S130 in which the value of the blood flow parameter for the vessel 110 is provided based on the calculated values of the blood flow parameter. This may be performed as described above, for example by calculating the provided value of the blood flow parameter as a weighted average of the individual values from the reconstructed images. This may also involve the identification of outlier values, and their exclusion from the weighted average, as also described above.

Alternative approaches may also be used to calculate a value of a blood flow parameter for the vessel 110 from the spectral attenuation data 120 in the operation S120. These may be performed in addition to, or instead of using a plurality of different image reconstruction techniques to provide a plurality of corresponding reconstructed images $130_{1..n}$, and calculating a value of the blood flow parameter from each reconstructed image, as described in the approach above.

In one example approach, in the operation S120, the received spectral attenuation data 120 is reconstructed to provide a reconstructed image $130_1$, and a plurality of values for the blood flow parameter are calculated from the image using a blood flow computation algorithm 140. A value of the blood flow parameter is calculated using different values for a set of model parameters $150_{1..i}$ of the blood flow computation algorithm.

In this example, the model parameters may include geometric parameters of the model and/or boundary conditions of the model. The blood flow computation algorithm may include a lumped parameter model, or a CFD model, as mentioned above. For example, if the model is a lumped parameter model, values of the blood flow parameter may be determined for the model using different values for a set of geometric parameters of the model, or using different values of a set of boundary conditions of the model. With reference to Fig. 4, geometric parameters for the model may be calculated from a reconstructed image, for example from a reconstructed image $130_1$. A set of model parameters $150_{1..i}$ may for instance represent a diameter of the vessel lumen at different positions along its length. Different values for the diameters may be determined by applying different thresholds to the image intensity values in the reconstructed image that are used to distinguish contrast agent in the vessel, a cross sectional area of which represents vessel lumen diameter, from the vessel wall. Alternatively, a different segmentation algorithm can be used. A set of model parameters $150_{1..i}$ may alternatively represent boundary conditions of the model such as pressure values at inlets and outlets of lumens in the vascular region. Different values for the boundary conditions may be provided by making different assumptions in the model regarding the actual pressure values. Such assumptions are detailed in the literature cited for each model. As an example, global overall pressure drop boundary condition could be varied to estimate the sensitivity of the model.

In another example approach, in the operation S120, the received spectral attenuation data 120 is reconstructed to provide a reconstructed image, a segmentation algorithm is applied to the reconstructed image, and a plurality of values for the blood flow parameter are calculated from the segmented image using a blood flow computation algorithm. A value of the blood flow parameter is calculated from a segmented image that is obtained by applying multiple different segmentation algorithms to the reconstructed image. Various different segmentation algorithms such as model-based segmentation, watershed-based segmentation, region growing or level sets or graphcuts may be used for this purpose. Alternatively, different parameter settings of a single algorithm can be used.

In another example approach, in the operation S120, a value of the blood flow parameter is calculated from the received spectral attenuation data 120 using each of a plurality of different blood flow computation algorithms.

In this example, the different blood flow computation algorithms may for instance be provided by different lumped parameter models, or different CFD models.

The above-mentioned example approaches may be used alone, or in combination, to calculate, using different techniques, a plurality of values of a blood flow parameter for the vessel 110 in the operation S120.

As mentioned above, in some examples, such as the example illustrated in Fig. 4, the operation of calculating S120 a value of a blood flow parameter for the vessel 110, is performed using a blood flow computation algorithm 140, and the blood flow computation algorithm 140 includes a lumped parameter model. A lumped parameter model includes a set of equations that represent blood flow in the model, and the equations are solved in order to calculate the value of a blood flow parameter.

In general, a lumped parameter model can be used to represent the blood flow within the vascular region as an electrical circuit. A volumetric flow rate of the blood in the vascular region is represented in the electrical circuit by an electrical current, and a pressure of the blood in the vascular region is represented in the electrical circuit by a voltage. A blood volume in the vascular region is represented by an electrical charge. As mentioned above, in a lumped parameter model, resistance to blood flow is represented by an electrical resistance, vessel wall compliance is represented by a capacitance, and blood inertia is represented by inductance. A machine learning-based approach for setting the values of resistors, capacitors, and indictors, in such a lumped parameter model that is based on angiographic geometric measurements of the vessels in the vascular region, is disclosed in the document by Nickisch, H., et al., cited above, and also in the document WO 2016/001017. Values for the blood flow rate and pressure in the model are then solved in order to determine a value for the FFR for a vessel. Another technique for providing, and determining the parameters of a lumped parameter model is described in the document by Kim, H. J., et al., cited above.

In more detail, as described in the document by Nickisch, H., et al., cited above, geometric measurements from a reconstructed angiographic image can be used to set the values of model parameters in the lumped parameter model. These model parameters include the values of the electrical components in the electrical circuit, and also the values of voltages or currents at nodes in the electrical circuit, i.e. boundary conditions. Such model parameters are determined from geometric measurements in the reconstructed image. Such measurements include for example the length, diameter, and curvature of lumens in the vascular region.

Fig. 6 is a schematic diagram illustrating an example of a lumped parameter model 140 including a set of model parameters $150_1$, in accordance with some aspects of the present disclosure. The lumped parameter model illustrated in Fig. 6 represents the branches in a vascular region as vessel segments, and the bifurcations in the vascular region are represented as nodes. As described in the document by Nickisch, H., et al., cited above, in general, the lumped parameter model includes two macroscopic component types: nonlinear vessel segment resistors (white tubes) and boundary conditions (black boxes). Fig. 6 is labelled with the boundary conditions for the vessel segments: "LCX" (left circumflex artery), "M1" (marginal artery branching off of the left circumflex artery), "LAD" (left anterior descending artery), "D1" (first diagonal artery), and so forth. A boundary condition may be a pressure or flow source driving the network; but any (lumped) boundary condition driving a conventional FE model can be used. The challenge is to translate the local geometry of the vessel (radius, perimeter, cross-sectional area) into parameters of the nonlinear resistor.

The hydraulic analogon to Ohm's law is Poiseuille's law stating that pressure drop $p$ and flow $f$ through a thin elongated pipe are linearly related by the linear transfer function $p_F$ so that $p = \varphi_P(f) = w_P f$, where the resistance constant wp depends on the length of the pipe and its cross-sectional area. Poiseuille friction is only one hydraulic effect causing a pressure drop (or energy loss) in hydraulic networks. Friction between blood and the vessel wall is caused by changes of cross-sectional area, bifurcations as well as ovality and curvature of the vessel. In the adopted framework illustrated in Table 1, a piecewise polynomial (invertible and point symmetric) effect transfer functions $\varphi_e^c(f) = w_e^c sign(f)|f|^{d_e}$ is used to model hydraulic effects with degree $d_e$ and geometry-specific weights $w_e^c$ depending solely on the local vessel geometry and material constants such as blood density r and viscosity m. If $d_e = 1$, we recover resistors and if $d_e \neq 1$, we talk about varistors. The functional form of the effect transfer functions represented below in Table 1 were taken from the fluid-mechanic literature where the individual hydraulic effects had been experimentally and analytically studied in isolation. To combine multiple interdependent effects $e = 1..E$, local-effect superposition $\varphi^c(f) = \sum_{e=1}^{E} \alpha_e \varphi_e^c(f)$ is assumed with effect-specific coefficients $\alpha_e$. An illustration of local-effect superposition and tree segment compression is given at the bottom of Table 1. In total, 6 different hydraulic effects are included, where the first 5 have a single coefficient each and the last one has three coefficients, yielding a total of 8 coefficients. To compress the size of the hydraulic network, the transfer functions $\varphi^c(f)$ is summed-up along a tree segment's centerline $c = 1..C$ into a single tree segment transfer function $\varphi(f) = \sum_{c=1}^{C} \varphi^c(f)$. This is possible because the flow through a tree segment is constant within the segment. The compression operation can be inverted by expansion, once the simulation is done and the value of $f$ is known. The hydraulic effects are also location specific in the sense that the first 5 effects are active everywhere except at bifurcations and the last one is active only at bifurcations. This representation of the hydraulic network as an assembly of transfer functions encoding hydraulic effects is then used to simulate blood flow.

| Hydraulic effect | Geometry, weights $w_e$, coefficients $a_e$ and degrees $d_e$ | | Pictogram |
|---|---|---|---|
| 1) Poiseuille friction | $w_P = 8\pi\mu \dfrac{l}{A^2}$ | $d_P = 1$ | |
| 2) Expansion friction | $w_E = \dfrac{\rho}{2} max^2\left(0, \dfrac{1}{A_{in}} - \dfrac{1}{A_{out}}\right)$ | $d_E = 2$ | |

(continued)

| Hydraulic effect | Geometry, weights $w_e$, coefficients $a_e$ and degrees $d_e$ | | Pictogram |
|---|---|---|---|
| 3) Ovality friction | $$w_O = w_P \cdot \left(\frac{p^2}{4\pi A}\right)^{\angle}$$ | $d_O = 1$ | |
| 4) Curvature friction | $$w_C = w_P \cdot max\left(0, \frac{19}{8}(rk)^{\frac{1}{25}} - 1\right)$$ | $d_C = 1$ | |
| 5) Bernoulli's principle | $$w_B = \frac{\rho}{2}\left(\frac{1}{A_{in}^2} - \frac{1}{A_{out}^2}\right)$$ | $d_B = 2$ | |
| 6) Bifurcation friction | $$w_{B_0} = \frac{\rho}{2A_{in}^2}, \; w_{B_1} = \frac{\rho}{2A_{out,1}^2}, \; , \; w_{B_2} = \frac{\rho}{2A_{out,2}^2}$$ | $d_{B0} = d_{B1} = d_{B2} = 2$ | |
| Local effect superposition | $$\varphi^c(f) = \sum_{e=1}^{E} \alpha_e \varphi_e^c(f)$$ | | |
| Tree segment compression | $$\varphi(f) = \sum_{c=1}^{C} \varphi^c(f).$$ | | |

Table 1: Transfer functions and hydraulic effects. The geometry weights are obtained from the vessel's local cross-sectional area $A$, perimeter $P$, length $l$, radius $r$ and curvature $\kappa$. Blood density is denoted by $r$ and viscosity by $m$.

Note that the cross-sections are not circular. Local effect-specific transfer functions $\varphi_e^c(f)$ are linearly combined to yield local transfer functions $\varphi^c(f)$. The local transfer functions $\varphi^c(f)$ are summed along the centerline $c = 1..C$ to yield the tree segment transfer function $\varphi(f)$.

A modified nodal analysis technique that is described in more detail in the document by Nickisch, H., et al. cited above, is then used to solve a circuit graph representation of the model in order to determine the values of the pressures and volumetric flow rates at each of the nodes in the model. The model parameters a in Table 1 are then learned by comparing the lumped model prediction with a CFD reference simulation. This results in values for the pressure, and the flow, at positions along the centerline of the vessel.

Thus, in examples in which the operation of calculating S120 a value of a blood flow parameter for the vessel 110 is performed using a lumped parameter model, the method described above with reference to Fig. 1 may also include:

extracting from each of the reconstructed images, model parameters 150 for a corresponding lumped parameter model representing a blood flow within the vascular region; and
wherein the calculating S120 a value of a blood flow parameter for the vessel 110, is performed using the corresponding lumped parameter model and model parameters 150.

In these examples, the operation of extracting model parameters 150 comprises:

segmenting the reconstructed image to provide a three-dimensional model representing the vascular region;
determining geometric data for the vascular region from the three-dimensional model; and
determining the values of the model parameters 150 from the geometric data.

In these examples, various known segmentation algorithms may be used to segment the reconstructed image. The geometric data that is determined may for example include dimensions of lumen(s) in the vascular region, a measure of curvature of the lumen(s) in the vascular region, a cross sectional area, and a shape of the lumen(s), and so forth, as described above with reference to Table 1. Image intensity values in the segmented image may also be used to provide values for the model parameters. For instance, a flow rate boundary condition can be determined based on the image intensity within a segmented vessel.

Returning to Fig.1, the value of the blood flow parameter that is provided in the operation S130 may be provided in various ways. In general, the value of the blood flow parameter is outputted. For example, the value of the blood flow parameter may be provided by outputting its value to a display device such as the monitor 260 of the system 200 illustrated in Fig. 2. Alternatively, the value of the blood flow parameter may be outputted to a computer readable storage medium, or to a printer device, for example. In some examples, the value of the blood flow parameter is outputted as a numerical value, and in other examples, the value of the blood flow parameter may be outputted graphically. For instance, the value of the blood flow parameter may be outputted as a color-coded icon. The value of the blood flow parameter may also be calculated at multiple positions along a length of the vessel and outputted graphically. For example, an image illustrating the vessel, or the vascular region, may be outputted with the values of the blood flow parameter displayed at corresponding positions along the length of the vessel as a color-coded overlay on the image. A physician may then use the provided value(s) of the blood flow parameter to inform a clinical diagnosis on a subject.

The inventors have also observed that despite the use of reconstructed spectral attenuation data to visualize stenoses in a vascular region, it can be difficult to convey to the physician some of the underlying anatomical causes of a stenosis in a reconstructed image. For example, if a vessel includes a stenosis, it is useful to convey to the physician the relative amounts of calcification, soft tissue, plaque, vessel, and lumen, in order to help the physician perform a diagnosis on the vessel. However, these quantities can represent a relatively small portion of a reconstructed image, which makes them difficult to identify. In response to this observation, in some examples, a contrast in the reconstructed image is adjusted based on the value of the blood flow parameter that is provided for the vessel. This has the effect of drawing the physician's attention to the anatomical causes of a clinically significant value of blood flow parameter.

In one example, the method described above with reference to Fig. 1 also includes:

generating S140, from the provided value of the blood flow parameter for the vessel 110, and an angiographic image reconstructed from the spectral attenuation data 120, or an angiographic image reconstructed from conventional X-ray attenuation data representing the vessel 110, a contrast-adjusted reconstructed image wherein a contrast in the reconstructed image is adjusted based on the value of the blood flow parameter provided for the vessel 110.

In this example, the contrast may be adjusted in the entire contrast-adjusted reconstructed image, or it may be adjusted only in a predetermined region 160 thereof. In the latter case, the contrast may be enhanced in a predefined region surrounding the position in the reconstructed angiographic image for which the provided value of the blood flow parameter meets a predetermined criterion. For instance, the contrast may be adjusted only within a circular region surrounding a position in the vessel if the value of the blood flow parameter is less than a threshold value at that position. By locally adjusting the contrast in this manner, a physician's attention is focused on features in the anatomical region surrounding the clinically-significant finding that the value of the blood flow parameter exceeds the threshold value, whilst leaving the appearance of the remainder of the reconstructed image unchanged. The contrast may for example be increased in the contrast-adjusted reconstructed image.

The contrast can be adjusted in various ways. For instance, a "windowing" technique, also known as grey-level mapping, contrast stretching, histogram modification or contrast enhancement, may be used. By way of an example, a so-called "level and window" approach may be used wherein values are set for a window width, i.e. a range of X-ray attenuation values that are to be mapped between greyscale black and greyscale white values, and for a window level, i.e. a center of the range of X-ray attenuation values. By setting the values of the window width and the window level, based on the value of the blood flow parameter, image intensity values in the reconstructed image may be mapped to new values in the contrast-adjusted reconstructed image. For instance, the brightness of the image in can be increased in the contrast-adjusted reconstructed image by decreasing the window level, thereby improving the visibility of anatomical features.

The angiographic image that is used to generate the contrast-adjusted angiographic image may be generated from the spectral attenuation data 120 that is received in the operation S110, or from conventional X-ray attenuation data representing the vessel 110. In the former case, the angiographic image may be a reconstructed image that is used to calculate a value of a blood flow parameter for the vessel 110 in the operation S120, or another image that is reconstructed from the spectral attenuation data. In either case, a decision over which of multiple image reconstruction technique to use to generate the angiographic image, or a decision over which of multiple windowing techniques to use in order to provide the contrast-adjusted image, may be made using a classification algorithm such as a random forest, or a support vector machine. The classification algorithm may decide which technique to use to adjust the contrast based on rules relating to e.g. the type of anatomical region represented in the image, the value of the blood flow parameter, and so forth. For instance, at a strong local pressure drop in the reconstructed image there is expected to be a lesion. Consequently, there is expected to be plaque, and a narrow contrast-filled vessel. In accordance with the rules, a predefined local region surrounding the pressure drop might be displayed in the contrast-adjusted image as purely a contrast agent image. The remainder of the contrast-adjusted image might be displayed as classical Hounsfield Unit X-ray attenuation values. An additional layer might also be provided within the predefined local region in which plaque properties are identified. For instance, a material image, such as a calcium image, might be provided in order to highlight the presence of plaque in the vicinity of the lesion. By way of another example, in an anatomical region with a relatively high level of perfusion, the contrast between perfused muscle and contrast-filled vessel will be different. In this anatomical region, the rules might provide that the material of the contrast agent is not displayed because there is too much contrast agent in the tissue.

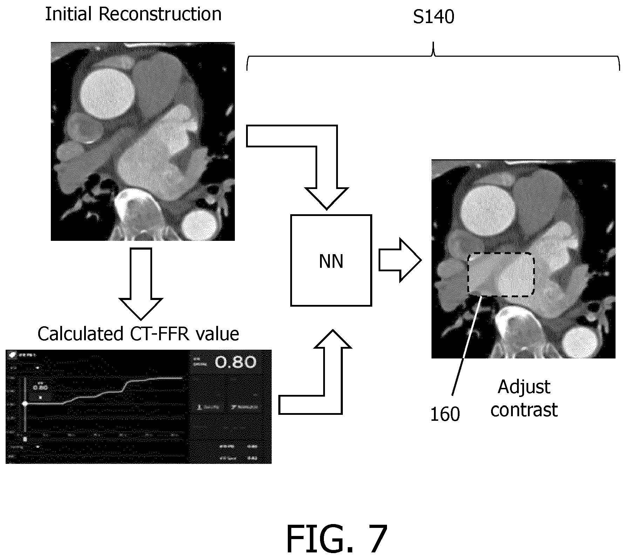

In one example, a neural network is used to adjust the contrast in the contrast-adjusted image. The neural network may adjust the contrast in a reconstructed image directly, or alternatively the neural network may be used to identify which of multiple reconstruction techniques, or which of multiple windowing techniques to apply, in order to provide the contrast-adjusted image. This example is described with reference to Fig. 7, which is a schematic diagram illustrating an example of an operation of generating S140 a contrast-adjusted reconstructed image, in accordance with some aspects of the present disclosure. In this example, the operation of generating a contrast-adjusted reconstructed image comprises:

inputting into a neural network the provided value of the blood flow parameter for the vessel 110, and the corresponding reconstructed image, or a corresponding angiographic image reconstructed from conventional X-ray attenuation data representing the vessel 110; and
wherein the neural network is trained to generate the contrast-adjusted reconstructed image from the inputted value of the blood flow parameter, and the corresponding reconstructed image.

In the example illustrated in Fig. 7, the blood flow parameter is an FFR value, and the contrast is adjusted only within a predetermined region 160 surrounding a position of the vessel at which the FFR value is less than a threshold value of 0.8. The neural network may be provided by various architectures, such as for example a convolutional neural network, "CNN", a recurrent neural network "RNN", or a transformer, and so forth.

The neural network NN illustrated in Fig. 7 may be trained to generate the contrast-adjusted reconstructed image by:

receiving training data, the training data comprising a plurality of volumetric training images representing the vascular region, and a corresponding value of a blood flow parameter for a vessel in the vascular region;
receiving ground truth data, the ground truth data comprising, for each of the volumetric training images, a corresponding ground truth contrast-adjusted reconstructed image wherein a contrast in the reconstructed image has been adjusted;
inputting the training data into the neural network; and
for each of a plurality of the inputted volumetric training images:

predicting, using the neural network, a corresponding contrast-adjusted reconstructed image;
adjusting parameters of the neural network based on a difference between the predicted contrast-adjusted reconstructed image and the ground truth contrast-adjusted reconstructed image; and
repeating the predicting and the adjusting, until a stopping criterion is met.

In this example, the volumetric training images may for instance be CT images that have been reconstructed from spectral CT data. The corresponding value of the blood flow parameter may have been determined using the techniques described above, or by invasive measurement in the vasculature, for example using a blood flow sensing wire, a pressure wire, or a Doppler ultrasound catheter. The ground truth data may be provided by an expert who has adjusted the contrast for the entire image, or for a predetermined region surrounding a portion of the vessel in which the blood flow parameter has clinical significance. If the neural network is trained to adjust the contrast directly, the ground truth data may include the contrast-adjusted image intensities. If the neural network is trained to identify which of multiple reconstruction techniques, or which of multiple windowing techniques to apply, in order to provide the contrast-adjusted image, the ground truth data may include an indication of a type of image reconstruction technique that should be used to generate the contrast-adjusted image, or an indication of the energy intervals that should be used to generate the contrast-adjusted image, or a type of windowing approach that should be used to generate the contrast-adjusted image.

In general, the training of a neural network involves inputting a training dataset into the neural network, and iteratively adjusting the neural network's parameters until the trained neural network provides an accurate output. Training is often performed using a Graphics Processing Unit "GPU" or a dedicated neural processor such as a Neural Processing Unit "NPU" or a Tensor Processing Unit "TPU". Training often employs a centralized approach wherein cloud-based or mainframe-based neural processors are used to train a neural network. Following its training with the training dataset, the trained neural network may be deployed to a device for analyzing new input data during inference. The processing requirements during inference are significantly less than those required during training, allowing the neural network to be deployed to a variety of systems such as laptop computers, tablets, mobile phones and so forth. Inference may for example be performed by a Central Processing Unit "CPU", a GPU, an NPU, a TPU, on a server, or in the cloud.

The process of training the neural network NN illustrated in Fig. 7 therefore includes adjusting its parameters. The parameters, or more particularly the weights and biases, control the operation of activation functions in the neural network. In supervised learning, the training process automatically adjusts the weights and the biases, such that when presented with the input data, the neural network accurately provides the corresponding expected output data. In order to do this, the value of the loss functions, or errors, are computed based on a difference between predicted output data and the expected output data. The value of the loss function may be computed using functions such as the negative log-likelihood loss, the mean absolute error (or L1 norm), the mean squared error, the root mean squared error (or L2 norm), the Huber loss, or the (binary) cross entropy loss. During training, the value of the loss function is typically minimized, and training is terminated when the value of the loss function satisfies a stopping criterion. Sometimes, training is terminated when the value of the loss function satisfies one or more of multiple criteria.

Various methods are known for solving the loss minimization problem such as gradient descent, Quasi-Newton methods, and so forth. Various algorithms have been developed to implement these methods and their variants including but not limited to Stochastic Gradient Descent "SGD", batch gradient descent, mini-batch gradient descent, Gauss-Newton, Levenberg Marquardt, Momentum, Adam, Nadam, Adagrad, Adadelta, RMSProp, and Adamax "optimizers" These algorithms compute the derivative of the loss function with respect to the model parameters using the chain rule. This process is called backpropagation since derivatives are computed starting at the last layer or output layer, moving toward the first layer or input layer. These derivatives inform the algorithm how the model parameters must be adjusted in order to minimize the error function. That is, adjustments to model parameters are made starting from the output layer and working backwards in the network until the input layer is reached. In a first training iteration, the initial weights and biases are often randomized. The neural network then predicts the output data, which is likewise, random. Backpropagation is then used to adjust the weights and the biases. The training process is performed iteratively by making adjustments to the weights and biases in each iteration. Training is terminated when the error, or difference between the predicted output data and the expected output data, is within an acceptable range for the training data, or for some validation data. Subsequently the neural network may be deployed, and the trained neural network makes predictions on new input data using the trained values of its parameters. If the training process was successful, the trained neural network accurately predicts the expected output data from the new input data.

In one example, patient data may also be inputted into the neural network NN described with reference to Fig. 7, and the contrast-adjusted angiographic image may be generated based also on the patient data. In this example, at inference, the operation of generating a contrast-adjusted reconstructed image comprises also includes:

receiving patient data relating to the vessel;
inputting the patient data into the neural network; and
generating, the contrast-adjusted angiographic image based further on the patient data; and
wherein the patient data comprises electronic health record data relating to the vessel and/or an identification of an implanted device in the vessel.

The patient data may include information pertaining to the patient such as a body mass index, smoking history, age, gender, data identifying an implanted device in the vessel, and so forth. In this example, the neural network is trained to trained to generate the contrast-adjusted angiographic image based further on the patient data. In so doing, the neural network may provide a contrast-adjusted image that is better-suited to the vessel in the reconstructed image.

The methods described above may be performed for multiple vessels, or for a selected vessel, in the vascular region. In the latter case, in one example, the method with reference to Fig. 1 further includes:

receiving input identifying the vessel 110 in the spectral attenuation data 120; and
wherein the providing S130 the value of the blood flow parameter for the vessel 110, is performed for the identified vessel.

The operation of identifying the vessel 110 may be performed in an image that is reconstructed from the spectral attenuation data that is received in the operation S110, or in an image that is reconstructed from conventional from conventional X-ray attenuation data. The vessel may be identified manually, or automatically. A manual identification of the vessel 110 may be performed by means of a user operating a user input device in combination with the displayed reconstructed image, for example. An automatic identification of the vessel 110 may be performed using a feature detector, or a trained neural network, for example. A segmentation may also be performed on the reconstructed image in order to assist in identifying the vessel. Various segmentation algorithms are known for this purpose. The feature detector, or the trained neural network, may automatically identify a candidate vessel in which to determine the value of the blood flow parameter. The vessel may be identified based on a type of the vessel, or based on the presence of an anomaly, such as a stenosis in the vessel. By way of an example, a feature detector, or a trained neural network may be used to identify the left coronary artery of the reconstructed image, and also a stenosis therein, and to identify the vessel as a candidate vessel for use in making a blood flow measurement such as the FFR. The blood flow measurements may subsequently be provided for the vessel at the position of the stenosis.

In another example, a computer program product, is provided. The computer program product comprises instructions which when executed by one or more processors, cause the one or more processors to carry out a method of determining a value of a blood flow parameter for a vessel 110, including:

receiving S110 spectral attenuation data 120 representing an injected contrast agent within a vascular region including the vessel 110, the spectral attenuation data defining X-ray attenuation within the vascular region in a plurality of different energy intervals $DE_{1..m}$;

calculating S120, from the spectral attenuation data 120, and using a plurality of different techniques, a value of a blood flow parameter for the vessel 110; and

providing S130 the value of the blood flow parameter for the vessel 110 based on the calculated values of the blood flow parameter.

In another example, a system 200 for determining a value of a blood flow parameter for a vessel 110, is provided. The system includes one or more processors 210 configured to:

receive S110 spectral attenuation data 120 representing an injected contrast agent within a vascular region including the vessel 110, the spectral attenuation data defining X-ray attenuation within the vascular region in a plurality of different energy intervals $DE_{1..m}$;

calculate S120, from the spectral attenuation data 120, and using a plurality of different techniques, a value of a blood flow parameter for the vessel 110; and

provide S130 the value of the blood flow parameter for the vessel 110 based on the calculated values of the blood flow parameter.

An example of the system 200 is illustrated in Fig. 2. It is noted that the system 200 may also include one or more of: a spectral X-ray imaging system for generating the spectral attenuation data 120 that is received in the operation S110, such as for example the spectral CT imaging system 220 illustrated in Fig. 2; a monitor 260 for displaying the provided value of the blood flow parameters, reconstructed images, and so forth; a patient bed 270; an injector (not illustrated in Fig. 2) for injecting a contrast agent into the vasculature; and a user input device configured to receive user input, such as a keyboard, a mouse, a touchscreen, and so forth.

The above examples are to be understood as illustrative of the present disclosure, and not restrictive. Further examples are also contemplated. For instance, the examples described in relation to computer-implemented methods, may also be provided by the computer program product, or by the computer-readable storage medium, or by the system 200, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

**Claims**

1. A computer-implemented method of determining a value of a blood flow parameter for a vessel (110), the method comprising:

receiving (S110) spectral attenuation data (120) representing an injected contrast agent within a vascular region including the vessel (110), the spectral attenuation data defining X-ray attenuation within the vascular region in a plurality of different energy intervals ($DE_{1..m}$);

calculating (S120), from the spectral attenuation data (120), and using a plurality of different techniques, a value of a blood flow parameter for the vessel (110); and

providing (S130) the value of the blood flow parameter for the vessel (110) based on the calculated values of the blood flow parameter.

2. The computer-implemented method according to claim 1, wherein the calculating (S120) a value of a blood flow parameter for the vessel (110) comprises one or more of:

> reconstructing the received spectral attenuation data (120) using a plurality of different image reconstruction techniques to provide a plurality of corresponding reconstructed images $(130_{1..n})$, and calculating a value of the blood flow parameter from each of the reconstructed images;
>
> reconstructing the received spectral attenuation data (120) to provide a reconstructed image $(130_1)$, and calculating a plurality of values for the blood flow parameter from the image using a blood flow computation algorithm (140), each value of the blood flow parameter being calculated using different values for a set of model parameters $(150_{1..i})$ of the blood flow computation algorithm;
>
> reconstructing the received spectral attenuation data (120) to provide a reconstructed image, applying a segmentation algorithm to the reconstructed image, and calculating a plurality of values for the blood flow parameter from the segmented image using a blood flow computation algorithm, each value of the blood flow parameter being calculated from a segmented image that is obtained by applying a different segmentation algorithm to the reconstructed image; and
>
> calculating a value of the blood flow parameter from the received spectral attenuation data (120) using each of a plurality of different blood flow computation algorithms.

3. The computer-implemented method according to claim 2, wherein the calculating (S120) a value of a blood flow parameter for the vessel (110) comprises reconstructing the received spectral attenuation data (120) using a plurality of different image reconstruction techniques to provide a plurality of corresponding reconstructed images, and calculating a value of the blood flow parameter from each of the reconstructed images; and wherein the reconstructed images are reconstructed by:

> applying different material decomposition algorithms to the spectral attenuation data (120); and/ or
>
> reconstructing spectral attenuation data (120) comprising different selections of the energy intervals $(DE_{1..m})$.

4. The computer-implemented method according to claim 3, wherein the method further comprises:

> extracting from each of the reconstructed images, model parameters (150) for a corresponding lumped parameter model representing a blood flow within the vascular region; and
>
> wherein the calculating (S120) a value of a blood flow parameter for the vessel (110), is performed using the corresponding lumped parameter model and the model parameters (150).

5. The computer-implemented method according to claim 4, wherein the extracting model parameters (150) comprises:

> segmenting the reconstructed image to provide a three-dimensional model representing the vascular region;
>
> determining geometric data for the vascular region from the three-dimensional model; and
>
> determining the values of the model parameters (150) from the geometric data.

6. The computer-implemented method according to any one of claims 4 - 5, wherein the lumped parameter model represents the blood flow within the vascular region as an electrical circuit; and wherein a volumetric flow rate of the blood in the vascular region is represented in the electrical circuit by an electrical current, and a pressure of the blood in the vascular region is represented in the electrical circuit by a voltage.

7. The computer-implemented method according to any one of claims 1 - 6, wherein the providing (S130) the value of the blood flow parameter for the vessel (110) based on the calculated values of the blood flow parameter comprises:

> analyzing the calculated values of the blood flow parameter to identify outlier values; and
>
> providing the value of the blood flow parameter for the vessel (110) as a weighted average of the calculated values that are not identified as outlier values.

8. The computer-implemented method according to claim 7, wherein the analyzing the calculated values of the blood flow parameter comprises performing a statistical analysis on the calculated values of the blood flow parameter.

9. The computer-implemented method according to any one of claims 1 - 8, wherein the method further comprises outputting a value representing a variation in the calculated values for the blood flow parameter.

10. The computer-implemented method according to any one of claims 1 - 9, wherein the calculating (S120) a value of a blood flow parameter for the vessel (110), and the providing (S130) the value of the blood flow parameter for the vessel (110), are performed at a plurality of positions along the vessel.

11. The computer-implemented method according to any one of claims 1 - 10, wherein the method further comprises: generating (S140), from the provided value of the blood flow parameter for the vessel (110), and an angiographic image reconstructed from the spectral attenuation data (120), or an angiographic image reconstructed from conventional X-ray attenuation data representing the vessel (110), a contrast-adjusted reconstructed image wherein a contrast in the reconstructed image is adjusted based on the value of the blood flow parameter provided for the vessel (110).

12. The computer-implemented method according to claim 11, wherein the generating a contrast-adjusted reconstructed image comprises:

inputting into a neural network the provided value of the blood flow parameter for the vessel (110), and the corresponding reconstructed image, or a corresponding angiographic image reconstructed from conventional X-ray attenuation data representing the vessel (110); and
wherein the neural network is trained to generate the contrast-adjusted reconstructed image from the inputted value of the blood flow parameter, and the corresponding reconstructed image.

13. The computer-implemented method according to claim 12, wherein the neural network is trained to generate the contrast-adjusted reconstructed image by:

receiving training data, the training data comprising a plurality of volumetric training images representing the vascular region, and a corresponding value of a blood flow parameter for a vessel in the vascular region;
receiving ground truth data, the ground truth data comprising, for each of the volumetric training images, a corresponding ground truth contrast-adjusted reconstructed image wherein a contrast in the reconstructed image has been adjusted;
inputting the training data into the neural network; and
for each of a plurality of the inputted volumetric training images:

predicting, using the neural network, a corresponding contrast-adjusted reconstructed image;
adjusting parameters of the neural network based on a difference between the predicted contrast-adjusted reconstructed image and the ground truth contrast-adjusted reconstructed image; and
repeating the predicting and the adjusting, until a stopping criterion is met.

14. The computer-implemented method according to any one of claims 11 - 13, wherein the contrast is adjusted only in a predetermined region (160) in the reconstructed image.

15. The computer-implemented method according to any previous claim, wherein the method further comprises:

receiving input identifying the vessel (110) in the spectral attenuation data (120); and
wherein the providing (S130) the value of the blood flow parameter for the vessel (110), is performed for the identified vessel.

FIG. 1

FIG. 2

FIG. 3

S120

120

Multi-channel
reconstructed images
$130_{1..n}$

140, $150_1$

140, $150_2$

140, $150_3$

Set of boundary conditions
$150_{1..i}$

S130

Variability
analysis,
averaging

CT-
FFR

FIG. 4

FIG. 5

FIG. 6

Initial Reconstruction

S140

Calculated CT-FFR value

NN

Adjust
160 contrast

# FIG. 7

Europäisches
Patentamt

European
Patent Office

Office européen
des brevets

# EUROPEAN SEARCH REPORT

Application Number

EP 22 18 6700

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/078619 A1 (HSIEH JIANG [US] ET AL) 17 March 2016 (2016-03-17) | 1,7-15 | INV. A61B6/00 G01V5/00 |
| A | * paragraphs [0100], [0099], [0159] * | 2-6 | |
| A | US 2022/148735 A1 (SHIGE FUMIMASA [JP]) 12 May 2022 (2022-05-12) * paragraphs [0068], [0117] – [0119] * | 2-6 | |
| A | US 2022/198655 A1 (GRIMMER RAINER [DE] ET AL) 23 June 2022 (2022-06-23) * paragraphs [0136] – [0138] * | 2-6 | |
| A | US 2021/219849 A1 (RUDIN STEPHEN [US] ET AL) 22 July 2021 (2021-07-22) * paragraphs [0038], [0049], [0075], [0085] * | 2-6 | |

TECHNICAL FIELDS
SEARCHED        (IPC)

A61B
G01N
G01V

The present search report has been drawn up for all claims

| Place of search | Date of completion of the search | Examiner |
|---|---|---|
| Munich | 30 November 2022 | Anscombe, Marcel |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
........................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 22 18 6700

30-11-2022

| Patent document cited in search report | | | Publication date | Patent family member(s) | | | Publication date |
|---|---|---|---|---|---|---|---|
| US 2016078619 | A1 | | 17-03-2016 | CN | 106687043 | A | 17-05-2017 |
| | | | | JP | 6680768 | B2 | 15-04-2020 |
| | | | | JP | 2017527383 | A | 21-09-2017 |
| | | | | KR | 20170054480 | A | 17-05-2017 |
| | | | | US | 2016078619 | A1 | 17-03-2016 |
| | | | | WO | 2016039891 | A1 | 17-03-2016 |
| US 2022148735 | A1 | | 12-05-2022 | DE | 102021129128 | A1 | 12-05-2022 |
| | | | | DE | 202021106122 | U1 | 08-03-2022 |
| | | | | JP | 2022076477 | A | 19-05-2022 |
| | | | | US | 2022148735 | A1 | 12-05-2022 |
| US 2022198655 | A1 | | 23-06-2022 | CN | 114649084 | A | 21-06-2022 |
| | | | | DE | 102020216306 | A1 | 23-06-2022 |
| | | | | US | 2022198655 | A1 | 23-06-2022 |
| US 2021219849 | A1 | | 22-07-2021 | JP | 2021112567 | A | 05-08-2021 |
| | | | | US | 2021219849 | A1 | 22-07-2021 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016001017 A

- WO 2007034359 A2

**Non-patent literature cited in the description**

- **BARFETT, J. J. et al.** Intra-vascular blood velocity and volumetric flow rate calculated from dynamic 4D CT angiography using a time-of-flight technique. *Int J Cardiovasc Imaging,* 2014, vol. 30, 1383-1392
- **PREVRHAL, S. et al.** CT Angiographic Measurement of Vascular Blood Flow Velocity by Using Projection Data. *Radiology,* December 2011, vol. 261 (3
- **TAYLOR. C. A. et al.** Computational Fluid Dynamics Applied to Cardiac Computed Tomography for Noninvasive Quantification of Fractional Flow Reserve: Scientific Basis. *JACC,* 2013, vol. 61 (22
- **KOO, B. K. et al.** Diagnosis of Ischemia-Causing Coronary Stenoses by Noninvasive Fractional Flow Reserve Computed From Coronary Computed Tomographic Angiograms: Results From the Prospective Multicenter DISCOVER-FLOW (Diagnosis of Ischemia-Causing Stenoses Obtained Via Noninvasive Fractional Flow Reserve) Study. *JACC,* 2011, vol. 58 (19

- **KIM, H. J. et al.** Patient-Specific Modeling of Blood Flow and Pressure in Human Coronary Arteries. *Annals of Biomedical Engineering,* October 2010, vol. 38 (10), 3195-3209
- **NICKISCH, H. et al.** Learning Patient-Specific Lumped Models for Interactive Coronary Blood Flow Simulations. *MICCAI 2015, Part II, LNCS,* 2015, vol. 9350, 433-441
- **BRENDEL, B. et al.** Empirical, projection-based basis-component decomposition method. *Medical Imaging 2009, Physics of Medical Imaging, edited by Ehsan Samei and Jiang Hsieh, Proc. of SPIE,* vol. 7258, 72583Y
- **ROESSL, E. ; PROKSA, R.** K-edge imaging in X-ray computed tomography using multi-bin photon counting detectors. *Phys Med Biol.,* 07 August 2007, vol. 52 (15), 4679-96
- **SILVA, A. C. et al.** Dual-energy (spectral) CT: applications in abdominal imaging. *RadioGraphics,* 2011, vol. 31 (4), 1031-1046